# EUROPEAN PATENT SPECIFICATION

(11) **EP 3 222 551 B1**
(45) Date of publication and mention of the grant of the patent: **22.12.2021**
(21) Application number: 17161530.5
(22) Date of filing: 17.03.2017
(51) Int. Cl.: B65D 51/28, A61B 10/00, B01L 3/00

(54) **CONTAINMENT DEVICE FOR BIOLOGICAL SAMPLES**
AUFNAHMEVORRICHTUNG FÜR BIOLOGISCHE PROBEN
DISPOSITIF DE CONFINEMENT POUR DES ÉCHANTILLONS BIOLOGIQUES

(30) Priority: 21.03.2016 IT UA20161845
(43) Date of publication of application: 27.09.2017
(73) Proprietor: Kaltek S.r.l., 35020 Saonara (PD) (IT)
(72) Inventor: CORTELAZZO, Lorenzo, 35133 PADOVA (IT)
(74) Representative: Modiano, Micaela Nadia

(56) References cited:
- US-A1- 2014 048 507
- US-A1- 2015 037 830
- US-A1- 2015 175 337
- US-A1- 2015 344 203
- US-B1- 9 132 950

## Description

The present invention relates to a containment device for biological samples.

Nowadays, after a surgical operation, any tissue samples removed may need to be sent to a laboratory for analysis. Usually in this step, the use is made of containers in which the biological sample is immersed in formaldehyde solutions (formalin), the use of which presents risks.

Formaldehyde, in fact, has recently passed from the classification "suspected of causing cancer" to the classification "may cause cancer", in the European Union regulations.

This has highlighted the problem of safety for analyst technicians, given the current impossibility of substituting formaldehyde with another, less problematic preservative.

Today containers are known and available on the market for samples of biological tissue that, although they use formaldehyde, set out to keep it isolated from contact with the technicians.

For example, EP2720617A1 discloses a container assembly which comprises a container for depositing a tissue sample, which has a bottom and a lid.

The lid comprises an upper element with a receptacle for a preservative, a seal for sealing the receptacle, and a sharp puncturing element that acts as a tool for breaking the seal by displacing the puncturing element.

The container has a first and a second position, wherein in the first position the container is isolated from the receptacle, and in the second position fluid can pass between the receptacle and the container.

The container comprises a separator arranged between the seal and the bottom of the container, and the separator is provided with at least one opening adapted to provide a passage of fluid between the receptacle and the container.

Such a container has a certain structural complexity, which has a negative influence on the cost of the product, and which also means the product is not easy to handle during use.

Another containment device can be found in US 2015/175337 A1, the document shows a containment device for biological samples, comprising: a first vessel for depositing a biological sample; a lid adapted to engage said first vessel, a hermetic chamber, adapted to contain a product, arranged between the lid and the first vessel,- means for generating a passage between said hermetic chamber and said first vessel for the descent of said product from said chamber to said first vessel, said means for generating a passage between said hermetic chamber and said first vessel comprising at least one region with a pre-fracture that is formed on said second vessel and a corresponding presser that extends from an insert in lid and is available for encountering said region with pre-fracture, the first vessel and the lid being mutually engaged with coupling means that define either a first configuration for use, in which the lid is closed and the second vessel is intact, or a second configuration for use, in which the lid is closed and is displaced toward the inside of the first vessel and the presser is arranged so as to interrupt the continuity of said second vessel at said region with pre-fracture.

In particular, during the manufacturing step, the seal needs to be sealed to the edges of the opening of the receptacle for the preservative liquid, for example by way of heat-sealing or adhesive bonding operations; such operations require apparatuses and processes that have a significant impact on the final calculation of costs and of production times.

The aim of the present invention is to provide a containment device for biological samples that is capable of overcoming the above mentioned drawbacks of the known art.

Within this aim, an object of the invention is to provide a containment device that is capable of reducing the number of components to the minimum.

Another object of the invention is to provide a containment device that is capable of maintaining a high level of safety in use.

Another object of the invention is to provide a containment device at low cost.

Another object of the invention is to provide a containment device that is simpler and quicker to assemble.

Another object of the invention is to provide a containment device that is easy and intuitive to use.

This aim and these and other objects which will become better apparent hereinafter are achieved by a containment device for biological samples according to claim 1.

Further characteristics and advantages of the invention will become better apparent from the detailed description of a particular, but not exclusive, embodiment of the containment device according to the invention, which is illustrated by way of non-limiting example in the accompanying drawings wherein:
Figure 1 is an exploded perspective view of a containment device according to the invention;
Figure 2 is a perspective view of a component of the device of Figure 1;
Figure 3 is a cross-sectional side view of the device according to the invention in a first configuration of use;
Figure 4 is the same side view as Figure 3 in a second configuration of use;
Figure 5 is an enlarged detail of the cross-section of Figure 3;
Figure 6 is a detail of a plan view of the component of Figure 2;
Figure 7 is a cross-sectional view of a detail of Figure 6;
Figure 8 is a cross-sectional side view of a variation of embodiment of the containment device according to the invention.

With reference to the figures, a containment device for biological samples according to the invention is generally designated with the reference numeral 10.

The containment device 10 comprises:
- a first vessel 11 for depositing a biological sample,
- a lid 12 adapted to engage the first vessel 11,
- a hermetically-sealed chamber 13, adapted to contain a preservative, arranged between the lid 12 and the first vessel 11,
- means of generating a passage between the hermetically-sealed chamber 13 and the first vessel 11 for the descent of the preservative from the chamber 13 to the first vessel 11.

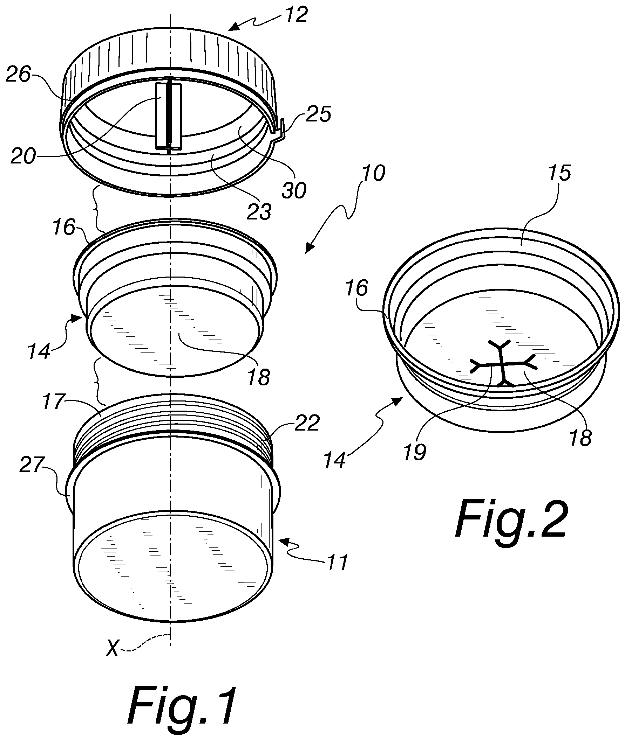

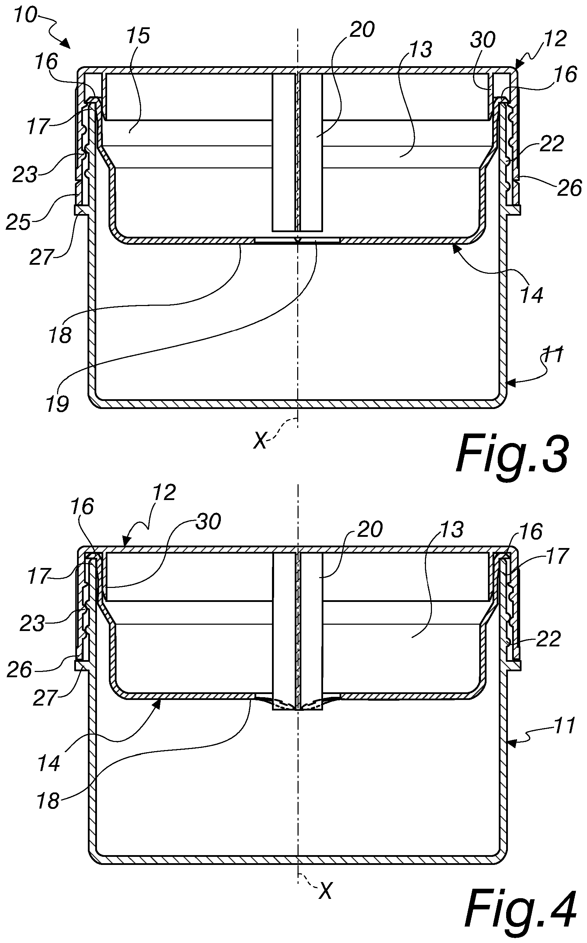

The peculiarity of the containment device 10 according to the invention consists in that:
- the hermetically-sealed chamber 13 is defined by a second vessel 14, which is inserted into the first vessel 11 with the filling opening 15 directed toward the lid 12, and by the lid 12 itself, closed onto the first vessel 11, as can clearly be seen in Figure 2; the second vessel 14 comprises an external perimetric shoulder 16 for resting on a corresponding supporting rim 17 of the first vessel 11;
- the means of generating a passage between the hermetically-sealed chamber 13 and the first vessel 11 comprise a region 18 with a pre-fracture 19 that is defined on the second vessel 14 and a corresponding presser 20 that extends from the lid 12 and is in a position to encounter the region 18 with a pre-fracture 19;
- the first vessel 11 and the lid 12 are mutually engaged with coupling means, described below, that define either a first configuration of use, in which the lid 12 is closed and the second vessel 14 is intact, as in Figure 3, or a second configuration of use, in which the lid 12 is closed and is displaced toward the inside of the first vessel 11 and the presser 20 is arranged so as to interrupt the continuity of the second vessel 14 at the region 18 with a pre-fracture 19, as in Figure 4.

The coupling means between the first vessel 11 and the lid 12 are constituted, in the present embodiment of the invention, by an external threading 22 around the inlet portion of the vessel 11 and by a corresponding internal threading 23 defined in the lid 12.

Closing the lid 12 on the first vessel 11 occurs therefore by screwing the external threading 22 with the internal threading 23.

A removable sealing portion 25 is present between the first vessel 11 and the lid 12 and is adapted to prevent the axial displacement of the lid 12 toward the inside of the first vessel 11.

The axial direction is designated by the axis X in the figures.

The removable portion 25 is constituted, for example, by a sealing ring arranged between the inlet rim 26 of the lid 12 and a corresponding external shoulder 27 of the first vessel 11.

In particular, the sealing ring is part of the lid 12, as can be seen in Figure 1.

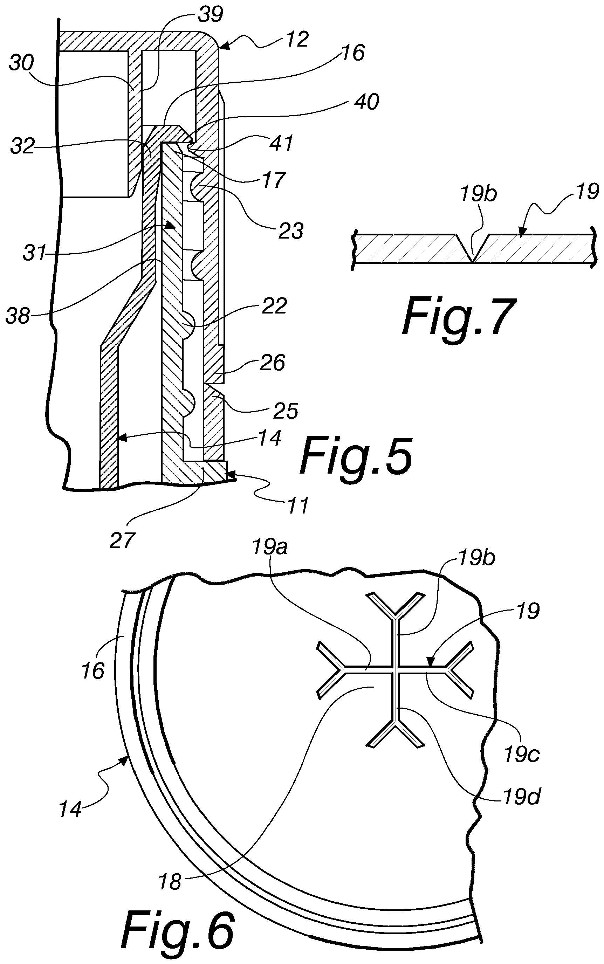

The lid 12 has a centering collar 30 that extends in the axial direction X, on the same side as the presser 20, and is adapted to pinch, between itself and the inlet portion 31 of the first vessel 11, an interposed sealing portion 32 of the second vessel 14, as shown clearly in Figure 5.

The sealing portion 32 is thicker than the radial distance between the mutually facing internal surface 38 of the inlet portion 31 and external surface 39 of the collar 30.

In this manner the sealing portion 32 is pressed between the inlet 31 and the collar 30, thus producing the seal of the chamber 13.

The containment device 10 according to the invention is provided, conveniently, with extraction-preventing fixing means between the lid 12 and the second vessel 14, which are designed to determine a stable fixing of the second vessel 14 to the lid 12 so that the removal of the lid 12 from the first vessel 11 does not entail the unwanted separation of the second vessel 14 from the lid 12, an event that, for a technician, would lead to evident risks of coming into contact with the formaldehyde.

In particular, in the present embodiment, the extraction-preventing fixing means are constituted by a perimetric radial protrusion 40, which extends from the external perimetric shoulder 16 in a radial direction until it passes beyond the supporting rim 17 in order to engage by snap action with an annular anti-extraction ridge 41 defined inside the lid 12.

Both the perimetric radial protrusion 40 and the annular anti-extraction ridge 41 are to be considered elastically deformable.

The presser 20 is constituted by a shank which is cross-shaped in cross-section and extends along the axis X of symmetry until it is proximate to the region 18 with pre-fracture regions 19.

The pre-fracture 19 comprises a series of lightening channels 19a, 19b, 19c, 19d, of which an illustrative cross-section is shown in Figure 7.

The first vessel 11, the lid 12 and the second vessel 14 are made of molded plastic material.

Preferably, the first vessel 11, the lid 12 and the second vessel 14 are made of recyclable and/or biodegradable polymer.

In particular, the first vessel 11, the lid 12 and the second vessel 14 are made of polypropylene (PP) or of high-density polyethylene (HDPE).

The containment device 10 according to the invention is therefore made up of only three elements:
- a first vessel 11,
- a lid 12,
- and a second vessel 14.

Assembly of the containment device 10 proceeds as follows.

The second vessel 14 is inserted into the first vessel 11, the second vessel 14 is filled with preservative, for example formaldehyde, and the containment device 10 is closed by screwing the lid 12 onto the first vessel 11, until the sealing ring of the lid 12 abuts against the external shoulder 27 of the first vessel 11, and until the extraction-preventing fixing means between the lid 12 and the second vessel 14 are engaged, i.e. until the perimetric radial protrusion 40 of the second vessel 14 is engaged by snap action with the internal annular ridge 41 of the lid 12.

The preservative is therefore in the chamber 13 defined between the lid 12 and the second vessel 14, hermetically sealed off from the external environment and from the risk of contact by a technician.

In order to place a biological sample in the first vessel 11, the lid 12 is unscrewed and is lifted off integrally with the second vessel 14.

After insertion of the biological sample into the first vessel 11, one proceeds to remove the portion of seal 25 and to close the lid 12 by screwing it to the first vessel 11.

During the screwing, at a certain point the presser 20 begins to press on the region 18 with a pre-fracture 19, and by continuing the screwing the region 18 ruptures and the preservative flows from the chamber 13 down into the first vessel 11, while remaining separated from contact with the operator.

With the containment device 10 completely closed, the sample is immersed in the preservative, which has always remained separated from the external environment.

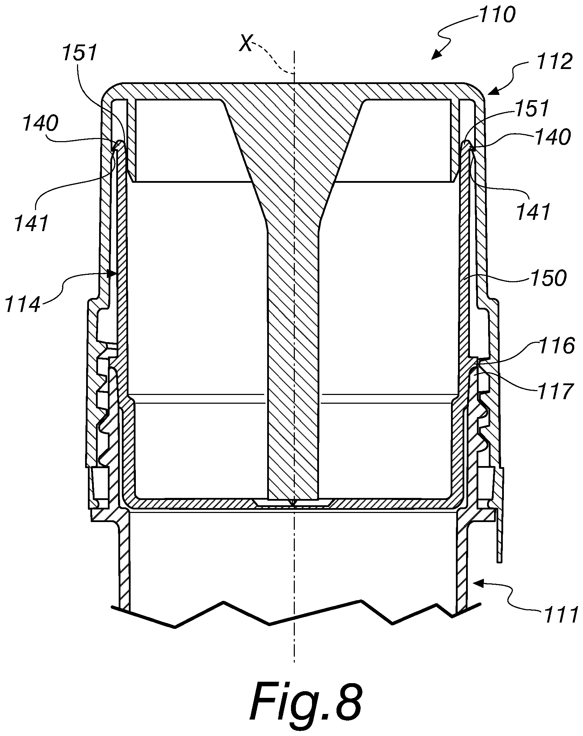

A variation of embodiment of the containment device according to the invention is shown in cross-section in Figure 8 and designated therein with the reference numeral 110.

In such variation of embodiment, the first vessel 111, the second vessel 114 and the lid 112 have a shape that extends predominantly axially with respect to an axis X.

In particular, the second vessel 114 has, above the external perimetric shoulder 116 for resting on a corresponding supporting rim 117 of the first vessel 111, a containment wall 150 that increases its containment capacity with respect to a second vessel 14 according to the embodiment described above.

At the free edge 151 of the containment wall 150 there is a perimetric external radial protrusion 140, which extends in a radial direction in order to engage by snap action with an annular anti-extraction ridge 141 defined inside the lid 112.

In practice it has been found that the intended aim and objects of the present invention have been achieved.

In fact, the containment device 10 devised overcomes the drawbacks of the known art, and reduces the number of components to the minimum, while maintaining a high level of safety in use.

Since the components preferably can be made of the same plastic material, for example recyclable and/or biodegradable polymer, problems associated with disposal are also avoided.

Furthermore, with the invention a containment device is provided which is low cost.

What is more, with the invention a containment device is provided that is simpler and quicker to assemble, there being no heat-sealing or adhesive bonding operation to perform, with corresponding gains in terms of components, equipment, labor, and production times.

Last but not least, with the invention a containment device is provided that is easy and intuitive both to assemble and to use.

The invention, thus conceived, is susceptible of numerous modifications and variations, all of which are within the scope of the appended claims. Moreover, all the details may be substituted by other, technically equivalent elements.

In practice the components and the materials employed, provided they are compatible with the specific use, and the contingent dimensions and shapes, may be any according to requirements and to the state of the art.

## Claims

1. A containment device (10) for biological samples, which comprises:
- a first vessel (11) for depositing a biological sample,
- a lid (12) adapted to engage said first vessel (11),
- a hermetically-sealed chamber (13), adapted to contain a preservative, arranged between the lid (12) and the first vessel (11),
- means of generating a passage between said hermetically-sealed chamber (13) and said first vessel (11) for the descent of said preservative from said chamber to said first vessel,
**characterized in that**:
- said hermetically-sealed chamber (13) is defined by a second vessel (14), which is inserted into said first vessel (11) with the filling opening (15) directed toward said lid (12), and by said lid (12) itself, closed onto said first vessel (11),
- said means of generating a passage between said hermetically-sealed chamber (13) and said first vessel (11) comprising at least one region (18) with a pre-fracture (19) that is defined on said second vessel (14) and a corresponding presser (20) that extends from said lid (12) and is in a position to encounter said region (18) with a pre-fracture (19),
- the first vessel (11) and the lid (12) being mutually engaged with coupling means that define either a first configuration of use, in which the lid (12) is closed and the second vessel (14) is intact, or a second configuration of use, in which the lid (12) is closed and is displaced toward the inside of the first vessel (11) and the presser (20) is arranged so as to interrupt the continuity of said second vessel (14) at said region (18) with a pre-fracture (19).

2. The containment device according to claim 1, **characterized in that** said coupling means between the first vessel (11) and the lid (12) are constituted by an external threading (22) around the inlet portion of said vessel (11) and by a corresponding internal threading (23) defined in said lid (12).

3. The containment device according to one or more of the preceding claims, **characterized in that** a removable sealing portion (25) is present between said first vessel (11) and said lid (12) and is adapted to prevent the axial displacement of said lid (12) toward the inside of said first vessel (11).

4. The containment device according to claim 3, **characterized in that** said removable portion (25) is constituted by a sealing ring arranged between the inlet rim (26) of said lid (12) and a corresponding external shoulder (27) of said first vessel (11).

5. The containment device according to claim 4, **characterized in that** said sealing ring is part of said lid (12).

6. The containment device according to one or more of the preceding claims, **characterized in that** said lid (12) has a centering collar (30) that extends in the axial direction (X), on the same side as said presser (20), and is adapted to pinch, between itself and the inlet portion (31) of said first vessel (11), an interposed sealing portion (32) of said second vessel (14).

7. The containment device according to claim 6, **characterized in that** said sealing portion (32) is thicker than the radial distance between the mutually facing internal surface (38) of said inlet portion (31) and external surface (39) of said collar (30).

8. The containment device according to one or more of the preceding claims, **characterized in that** it is provided with extraction-preventing fixing means between the lid (12) and the second vessel (14), which are designed to determine a stable fixing of the second vessel (14) to the lid (12).

9. The containment device according to one or more of the preceding claims, **characterized in that** said first vessel (11), said lid (12) and said second vessel (14) are made of molded plastic material.

10. The containment device according to one or more of the preceding claims, **characterized in that** said first vessel (11), said lid (12) and said second vessel (14) are made of recyclable and/or biodegradable polymer.

11. The containment device according to one or more of the preceding claims, **characterized in that** said second vessel (14) comprises an external perimetric shoulder (16) for resting on a corresponding supporting rim (17) of said first vessel (11).

## Patentansprüche

1. Eine Aufnahmevorrichtung (10) für biologische Proben, die Folgendes umfasst:
- ein erstes Gefäß (11) zum Eintragen einer biologischen Probe,
- einen Deckel (12), ausgebildet, um in Eingriff mit dem ersten Gefäß (11) zu stehen,
- eine hermetisch abgedichtete Kammer (13), ausgebildet, um einen Konservierungsstoff aufzunehmen, angeordnet zwischen dem Deckel (12) und dem ersten Gefäß (11),
- Mittel zum Erzeugen eines Durchgangs zwischen der hermetisch abgedichteten Kammer (13) und dem ersten Gefäß (11) für das Herabfließen des Konservierungsstoffs von der Kammer in das erste Gefäß;
**dadurch gekennzeichnet, dass**
- die hermetisch abgedichtete Kammer (13) durch ein zweites Gefäß (14) bestimmt ist, das in das erste Gefäß (11) eingesetzt ist, mit der Füllöffnung (15) dem Deckel (12) zugewandt, und durch den Deckel (12) selbst, der auf das erste Gefäß (11) aufgesetzt ist,
- wobei die Mittel zum Erzeugen eines Durchgangs zwischen der hermetisch abgedichteten Kammer (13) und dem ersten Gefäß (11) mindestens einen Bereich (18) mit einer Sollbruchstelle (19) umfassen, die an dem zweiten Gefäß (14) bestimmt ist, und eine entsprechende Druckvorrichtung (20), die sich von dem Deckel (12) erstreckt und sich in einer Position befindet, um in Kontakt mit dem Bereich (18) mit einer Sollbruchstelle (19) zu kommen,
- wobei das erste Gefäß (11) und der Deckel (12) miteinander über Kopplungsmittel in Eingriff stehen, die entweder eine erste Nutzungskonfiguration bestimmen, in welcher der Deckel (12) geschlossen ist und das zweite Gefäß (14) intakt ist, oder eine zweite Nutzungskonfiguration, in welcher der Deckel (12) geschlossen und zur Innenseite des ersten Gefäßes (11) verschoben ist und die Druckvorrichtung (20) angeordnet ist, um die Kontinuität des zweiten Gefäßes (14) in dem Bereich (18) mit Sollbruchstelle (19) zu unterbrechen.

2. Die Aufnahmevorrichtung gemäß Anspruch 1, **dadurch gekennzeichnet, dass** die Kopplungsmittel zwischen dem ersten Gefäß (11) und dem Deckel (12) in einem Außengewinde (22) um den Einlassabschnitt des Gefäßes (11) und einem entsprechenden Innengewinde (23) bestehen, das in dem Deckel (12) bestimmt ist.

3. Die Aufnahmevorrichtung gemäß einem oder mehreren der obigen Ansprüche, **dadurch gekennzeichnet, dass** ein abnehmbarer Dichtungsabschnitt (25) zwischen dem ersten Gefäß (11) und dem Deckel (12) vorhanden und ausgebildet ist, um die Axialverschiebung des Deckels (12) zur Innenseite des ersten Gefäßes (11) zu verhindern.

4. Die Aufnahmevorrichtung gemäß Anspruch 3, **dadurch gekennzeichnet, dass** der abnehmbare Abschnitt (25) aus einem Dichtring besteht, der zwischen dem Einlassrand (26) des Deckels (12) und einer entsprechenden äußeren Schulter (27) des ersten Gefäßes (11) angeordnet ist.

5. Die Aufnahmevorrichtung gemäß Anspruch 4, **dadurch gekennzeichnet, dass** der Dichtring Teil des Deckels (12) ist.

6. Die Aufnahmevorrichtung gemäß einem oder mehreren der obigen Ansprüche, **dadurch gekennzeichnet, dass** der Deckel (12) eine Zentrierschulter (30) hat, die sich auf derselben Seite wie die Druckvorrichtung (20) in die axiale Richtung (X) erstreckt und ausgebildet ist, um zwischen sich und dem Einlassabschnitt (31) des ersten Gefäßes (11) einen dazwischen angeordneten Abdichtungsabschnitt (32) des zweiten Gefäßes (14) einzuklemmen.

7. Die Aufnahmevorrichtung gemäß Anspruch 6, **dadurch gekennzeichnet, dass** der Abdichtungsabschnitt (32) dicker ist als der radiale Abstand zwischen der einander zugewandten inneren Oberfläche (38) des Einlassabschnitts (31) und äußeren Oberfläche (39) der Schulter (30).

8. Die Aufnahmevorrichtung gemäß einem oder mehreren der obigen Ansprüche, **dadurch gekennzeichnet, dass** sie mit das Herausziehen verhindernden Befestigungsmitteln zwischen dem Deckel (12) und dem zweiten Gefäß (14) ausgestattet ist, die dazu dienen, eine stabile Fixierung des zweiten Gefäßes (14) am Deckel (12) zu bestimmen.

9. Die Aufnahmevorrichtung gemäß einem oder mehreren der obigen Ansprüche, **dadurch gekennzeichnet, dass** das erste Gefäß (11), der Deckel (12) und das zweite Gefäß (14) aus geformtem Kunststoffmaterial bestehen.

10. Die Aufnahmevorrichtung gemäß einem oder mehreren der obigen Ansprüche, **dadurch gekennzeichnet, dass** das erste Gefäß (11), der Deckel (12) und das zweite Gefäß (14) aus recycelbarem und/oder biologisch abbaubarem Polymer hergestellt sind.

11. Die Aufnahmevorrichtung gemäß einem oder mehreren der obigen Ansprüche, **dadurch gekennzeichnet, dass** das zweite Gefäß (14) eine äußere Umfangsschulter (16) zum Aufliegen auf einem entsprechenden Stützrand (17) des ersten Gefäßes (11) umfasst.

## Revendications

1. Dispositif de confinement (10) pour des échantillons biologiques, qui comprend :
un premier récipient (11) pour déposer un échantillon biologique ;
un couvercle (12) adapté pour venir en prise avec le premier récipient (11) ;
une chambre hermétiquement scellée (13), adaptée pour contenir un conservateur, disposée entre le couvercle (12) et le premier récipient (11),
des moyens pour générer un passage entre ladite chambre hermétiquement scellée (13) et ledit premier récipient (11) pour la descente dudit conservateur depuis ladite chambre jusque dans ledit premier récipient,
**caractérisé en ce que** :
- ladite chambre hermétiquement scellée (13) est définie par un second récipient (14), qui est inséré dans ledit premier récipient (11) avec l'ouverture de remplissage (15) dirigée vers ledit couvercle (12), et par ledit couvercle (12) lui-même, fermé sur ledit premier récipient (11),
- lesdits moyens pour générer un passage entre ladite chambre hermétiquement scellée (13) et ledit premier récipient (11) comprenant au moins une région (18) avec une pré-fracture (19) qui est définie sur ledit second récipient (14) et un élément presseur correspondant (20) qui s'étend depuis ledit couvercle (12) et est dans une position permettant de rencontrer ladite région (18) avec une pré-fracture (19),
- le premier récipient (11) et le couvercle (12) étant mutuellement en prise avec des moyens d'accouplement qui définissent soit une première configuration d'utilisation, dans laquelle le couvercle (12) est fermé et le second récipient (14) est intact, soit une seconde configuration d'utilisation, dans laquelle le couvercle (12) est fermé et est déplacé vers l'intérieur du premier récipient (11) et l'élément presseur (20) est disposé de manière à interrompre la continuité dudit second récipient (14) au niveau de ladite région (18) avec une pré-fracture (19).

2. Dispositif de confinement selon la revendication 1, **caractérisé en ce que** lesdits moyens d'accouplement entre le premier récipient (11) et le couvercle (12) sont constitués par un filetage externe (22) autour de la partie d'entrée dudit récipient (11) et par un filetage interne correspondant (23) défini dans ledit couvercle (12).

3. Dispositif de confinement selon l'une ou plusieurs des précédentes revendications, **caractérisé en ce qu'**une partie d'étanchéité détachable (25) est présente entre ledit premier récipient (11) et ledit couvercle (12) et est adaptée pour empêcher le déplacement axial dudit couvercle (12) vers l'intérieur dudit premier récipient (11).

4. Dispositif de confinement selon la revendication 3, **caractérisé en ce que** ladite partie détachable (25) est constituée par une bague d'étanchéité disposée entre le rebord d'entrée (26) dudit couvercle (12) et un épaulement externe correspondant (27) dudit premier récipient (11).

5. Dispositif de confinement selon la revendication 4, **caractérisé en ce que** ladite bague d'étanchéité fait partie dudit couvercle (12).

6. Dispositif de confinement selon l'une ou plusieurs des précédentes revendications, **caractérisé en ce que** ledit couvercle (12) a un collier de centrage (30) qui s'étend dans la direction axiale (X), du même côté que ledit élément presseur (20), et est adapté pour pincer, entre lui-même et la partie d'entrée (31) dudit premier récipient (11), une partie d'étanchéité intercalée (32) dudit second récipient (14).

7. Dispositif de confinement selon la revendication 6, **caractérisé en ce que** ladite partie d'étanchéité (32) est plus épaisse que la distance radiale entre la surface interne (38) de ladite partie d'entrée (31) et la surface externe (39) dudit collier (30) qi se font mutuellement face.

8. Dispositif de confinement selon l'une ou plusieurs des précédentes revendications, **caractérisé en ce que** sont prévus des moyens de fixation, empêchant l'extraction, entre le couvercle (12) et le second récipient (14), qui sont adaptés pour déterminer une fixation stable du second récipient (14) au couvercle (12).

9. Dispositif de confinement selon l'une ou plusieurs des précédentes revendications, **caractérisé en ce que** ledit premier récipient (11), ledit couvercle (12) et ledit second récipient (14) sont faits d'une matière plastique moulée.

10. Dispositif de confinement selon l'une ou plusieurs des précédentes revendications, **caractérisé en ce que** ledit premier récipient (11), ledit couvercle (12) et ledit second récipient (14) sont faits d'un polymère recyclable et/ou biodégradable.

11. Dispositif de confinement selon l'une ou plusieurs des précédentes revendications, **caractérisé en ce que** ledit second récipient (14) comprend un épaulement périmétrique externe (16) pour reposer sur un rebord de support correspondant (17) dudit premier récipient (11).
